Europäisches Patentamt

European Patent Office

Office européen des brevets

(11) Veröffentlichungsnummer: **0 035 142**
A2

(12) **EUROPÄISCHE PATENTANMELDUNG**

(21) Anmeldenummer: **81100956.2**

(22) Anmeldetag: **11.02.81**

(51) Int. Cl.³: **A 61 K 9/10,** A 61 K 7/48,
A 61 K 7/40

(30) Priorität: **22.02.80 DE 3006635**

(43) Veröffentlichungstag der Anmeldung: **09.09.81**
**Patentblatt 81/36**

(84) Benannte Vertragsstaaten: **CH DE FR GB IT LI**

(71) Anmelder: **BASF Aktiengesellschaft,**
**Carl-Bosch-Strasse 38, D-6700 Ludwigshafen (DE)**

(72) Erfinder: **Buehler, Volker, Dr., In den Weingaerten 14,**
**D-6706 Wachenheim (DE)**

(54) **Verwendung von mikronisiertem vernetztem, unlöslichem Polyvinylpyrrolidon zur Stabilisierung von Suspensionen.**

(57) Die Erfindung betrifft die Verwendung von mikronisiertem vernetztem, unlöslichem Polyvinylpyrrolidon zur Stabilisierung von Suspensionen, insbesondere für pharmazeutische und kosmetische Zwecke.

EP 0 035 142 A2

Verwendung von mikronisiertem vernetztem, unlöslichem
Polyvinylpyrrolidon zur Stabilisierung von Suspensionen

Die Erfindung betrifft die Verwendung von mikronisiertem
vernetztem, unlöslichem Polyvinylpyrrolidon zur Stabilisierung von Suspensionen, insbesondere für pharmazeutische und kosmetische Zwecke.

Bei den Darreichungsformen eines Arzneimittels spielt die
Genauigkeit der Dosierung eine große Rolle, weshalb vordosierte Arzneimittelmengen, beispielsweise in Form von
Tabletten, Kapseln, Dragees oder Suppositorien, am häufigsten verwendet werden.

Bei der Anwendung flüssiger, oraler Arzneiformen werden in
der Regel Dosierungsangaben in Tropfen, Tee-, Eß- oder
Meßlöffeln gemacht. Diese Angaben setzen als selbstverständlich voraus, daß der Wirkstoff in der flüssigen
Arzneiform völlig homogen verteilt ist oder vor der Entnahme homogenisiert wird, z.B. durch Schütteln. Bei klaren
Wirkstofflösungen ergeben sich keine Schwierigkeiten. Da
aber viele der heute üblichen Arzneistoffe schwer wasserlöslich sind und viele Lösungsmittel physiologisch
nicht unbedenklich sind, muß immer wieder auf die Suspension als Darreichungsform zurückgegriffen werden.

Die Suspension eines schwerlöslichen Arzneistoffes stellt
in der Regel ein grobdisperses System dar, von dem praktisch immer eine Phasentrennung zu erwarten ist und damit
auch eine Einschränkung der genauen Dosierungsmöglichkeit
bei der Anwendung. Um dies zu verhindern, bzw. eine Phasentrennung zu verzögern, werden Suspensionsvermittler, z.B.
Tenside, oder Suspensionsstabilisatoren verwendet. Letzere
können beispielsweise lösliche Polymere, wie Cellulose-

D/BL

C035142

derivate, Polyethylenglykole oder Polyvinylpyrrolidon, oder auch unlösliche Stoffe, wie Bentonit oder hochdisperses Siliciumdioxid, sein. Nicht in jedem Falle können diese Stabilisationshilfsmittel den gestellten Forderungen gerecht werden (vgl. Voigt, Lehrbuch der pharmazeutischen Technologie, Verlag Chemie 1975). So ist z.B. in einer Suspension von 5 % Chloramphenicol mit 5 % hochdispersem Siliciumdioxid die Verteilung des Wirkstoffes nach 15 Minuten derart, daß die Konzentration im untersten Teil der Suspension doppelt so hoch ist wie die im oberen Bereich.

Der vorliegenden Erfindung liegt die Aufgabe zugrunde, einen Suspensionsstabilisator zu finden, der den Anforderungen an eine Suspension im Hinblick auf das Sedimentationsverhalten, die Aufschüttelbarkeit, die Dosierungsgenauigkeit und die physiologische Verträglichkeit besser gerecht wird wie die üblicherweise verwendeten Suspensionshilfsmittel.

Gegenstand der Erfindung ist die Verwendung von mikronisiertem vernetztem, unlöslichem Polyvinylpyrrolidon als Hilfsmittel zur Herstellung stabiler Suspensionen für pharmazeutische und kosmetische Zwecke.

Mit dem erfindungsgemäß zu verwendenden Suspensionsstabilisator können ohne Schwierigkeiten Suspensionen von Arzneistoffen für die innerliche oder äußerliche Applikationsweise hergestellt werden, deren galenische Eigenschaften in hervorragender Weise die Anforderungen an eine Suspension als pharmazeutisches oder kosmetisches Fertigpräparat erfüllen, d.h. der Suspensionsquotient (Sedimentvolumen/Gesamtvolumen) muß nach 24 Stunden größer als 0,9 sein und eine ausreichende Aufschüttelbarkeit und gute Dosierungsgenauigkeit müssen nach 24 Stunden gewährleistet

C035142

sein (vgl. Ritschel et al., Österr. Apoth. Ztg. 15 (1961) 436).

Das erfindungsgemäß zu verwendende vernetzte unlösliche PVP kann beispielsweise nach DAC 1979 (Monographie "Unlösliches Polyvinylpyrrolidon") chrakterisiert werden. Vernetztes, unlösliches Polyvinylpyrrolidon ist eine in allen üblichen Lösungsmitteln unlösliche Substanz, deren Infrarot-Spektrum mit dem des löslichen Polyvinylpyrrolidons übereinstimmt. Es ist chemisch indifferent und physiologisch sehr gut verträglich.

Die Herstellung der mikronisierten Formen erfolgt in an sich üblicher Weise , beispielsweise durch Luftstrahlmahlung. Die Kornverteilung des mikronisierten Produktes ist derart, daß mindestens 99 % kleiner als 20 um, bzw. mindestens 80 % kleiner als 10 um sind.

Die als Suspensionsstabilisator verwendeten Mengen liegen bei oralen Suspensionen zwischen 1 Gew.% als untere Grenze und der Feststoffkonzentration, bei der ein freies Fließen der Suspension nicht mehr gegeben ist, als obere Grenze. Bei einer wäßrigen Suspension ohne Wirkstoff liegt diese Konzentration zwischen 17 und 20 %. Für pharmazeutische Suspensionen ergeben sich daraus Mengen von 1 bis 20 Gew.%.

Bei äußerlich anzuwendenden Suspensionen können die verwendeten Mengen auch höher sein, bis zu 30 Gew.%, wenn beispielsweise pastenartige Suspensionen erhalten werden sollen.

Die Suspensionen selbst werden in üblicher Weise durch mechanisches Dispergieren des mikronisierten vernetzten, unlöslichen Polyvinylpyrrolidons und der verwendeten Wirkstoffe in dem jeweils verwendeten Lösungsmittel durch-

C035142

geführt. Die Anwendung kann pharmazeutischer oder kosmetischer Art sein.

Als Lösungsmittel kommen insbesondere Wasser, Mischungen von Wasser mit Ethanol, Propanol, i-Propanol, Propylenglykol, Glycerin oder Polyethylenglykol sowie flüssiges Paraffin oder Neutralöl in Betracht.

Als geeignete Arzneimittelwirkstoffe kommen beispielsweise in Betracht:

Sulfonamide und andere Chemotherapeutika, wie Sulfamoxol, Sulfamethoxazin, Trimethoprim; Antibiotika, wie Chloramphenikol, Ampicillin; Hypnotika, wie Phenobarbital; Antiepileptika, wie Diphenylhydantoin; Antimycotika, wie Griseofulvin; Corticoide, wie Dexamethason; Urologika, wie Nalidixinsäure; Magen-Darm--Mittel, wie Kaolin.

Für kosmetische Zwecke sind insbesondere geeignet Zinkoxid, Titandioxid, Kaolin oder Strontiumsulfid.

Als zusätzliche Hilfsstoffe können lösliche Suspensionsstabilisatoren verwendet werden, z.B. lösliches Polyvinylpyrrolidon mit einem K-Wert von 85 bis 95. Dabei erhöht sich die Viskosität der Suspension, wodurch die notwendige Menge an mikronisiertem vernetztem, unlöslichem Polyvinylpyrrolidon ggfs. herabgesetzt werden kann. Auch ist der Zusatz von Geschmackstoffen, Antioxidantien, Konservierungsstoffen usw. in der üblichen Weise möglich.

Grundsätzlich sei festgestellt, daß der Fachmann die galenischen Eigenschaften eines festen Suspensionsstabilisators nicht generell vorauszusagen vermag. So ist es nicht vorhersehbar, daß eine 5 %ige Suspension von hoch-

C035142

dispersem Siliziumdioxid, die etwa die gleiche Viskosität wie eine 10 %ige Suspension von mikronisiertem vernetztem, unlöslichem Polyvinylpyrrolidon hat, (ca. 6 mPas, Rotationsviskosimeter) nach 24 Stunden ein Suspensionsquotienten von unter 0,8 aufweist, wogegen derjenige einer erfindungsgemäßen Suspension mit gleicher Viskosität nach 24 Stunden bei 0,96 liegt. Die erfindungsgemäße Suspension erfüllt die Anforderung an eine Suspension als Fertigpräparat, das Präparat mit hochdispersem Siliciumdioxid nicht. Im übrigen haben ausgiebige toxikologische Untersuchungen ergeben, daß vernetztes, unlösliches Polyvinylpyrrolidon praktisch untoxisch ist.

Beispiel 1

5 g Diphenylhydantoin und 10 g mikronisiertes vernetztes, unlösliches Polyvinylpyrrolidon werden in 100 ml Wasser suspendiert und gut geschüttelt. Man erhält eine weiße Suspension. Die Verteilung des Diphenylhydantoins in der Suspension ist nach 24 Stunden noch gleichmäßig.

Beispiel 2

5 g Trimethoprim und 10 g mikronisiertes vernetztes, unlösliches Polyvinylpyrrolidon werden in 100 ml Wasser suspendiert und gut geschüttelt. Die Verteilung des Trimethoprims in der Suspension ist über die Dauer von 1 Stunde gleichmäßig und die Aufschüttelbarkeit ist nach 3 Tagen immer noch gut.

Beispiel 3

1 g lösliches Polyvinylpyrrolidon mit einem K-Wert von 85 bis 95 werden in 100 ml Wasser gelöst. 5 g Diphenylhydantoin und 8 g mikronisiertes vernetztes, unlösliches

C035142

Polyvinylpyrrolidon werden in dieser Lösung suspendiert und gut geschüttelt. Man erhält eine idealviskose, weiße Suspension, in der das Diphenylhydantoin nach 24 Stunden Stehenlassen noch gleichmäßig verteilt ist.

Beispiel 4

10 g Zinkoxid und 8 g mikronisiertes vernetztes, unlösliches Polyvinylpyrrolidon werden in 100 ml Wasser suspendiert und gut geschüttelt. In dieser Suspension ist über die Dauer von mehr als 1 Stunde keine Sedimentation zu beobachten und die Verteilung der suspendierten Teile ist über diesen Zeitraum homogen.

Beispiel 5

10 g Zinkoxid und 5 g mikronisiertes vernetztes, unlösliches Polyvinylpyrrolidon werden in 100 ml dünnflüssigem Paraffin suspendiert und gut geschüttelt. Über einen Zeitraum von 1 Stunde ist in dieser Suspension keine Sedimentation zu beobachten.

C035142

Patentansprüche

1. Verwendung von mikronisiertem vernetztem, unlöslichem Polyvinylpyrrolidon als Hilfsmittel zur Herstellung stabiler Suspensionen.

2. Verwendung von mikronisiertem vernetztem, unlöslichem Polyvinylpyrrolidon in einer Menge von 1 bis 30 Gew.% zur Herstellung stabiler Suspensionen für pharmazeutische und kosmetische Zwecke.

3. Verfahren zur Herstellung von stabilen Suspensionen, dadurch gekennzeichnet, daß man mikroinisiertes vernetztes, unlösliches Polyvinylpyrrolidon als Suspensionshilfsmittel verwendet.